# EUROPEAN PATENT SPECIFICATION

(11) **EP 4 066 864 B1**
(45) Date of publication and mention of the grant of the patent: **06.08.2025**
(21) Application number: 22020141.2
(22) Date of filing: 29.03.2022
(51) Int. Cl.: A61L 2/10, G06F 3/00

(54) **APPARATUS FOR DISINFECTING HANDHELD ELECTRONIC DEVICE AND METHOD OF OPERATING THE SAME**
GERÄT ZUR DESINFEKTION EINER TRAGBAREN ELEKTRONISCHEN VORRICHTUNG UND BETRIEBSVERFAHREN DAFÜR
APPAREIL DE DÉSINFECTION DE DISPOSITIF ÉLECTRONIQUE PORTABLE ET SON PROCÉDÉ DE FONCTIONNEMENT

(30) Priority: 29.03.2021 US 202163167376 P
(43) Date of publication of application: 05.10.2022
(73) Proprietor: ReadyDock, Inc., Avon, CT 06001 (US)
(72) Inventor: DAIGLE, Michael, Glastonbury, Connecticut 06033 (US); ALLEN, Chris, West Hartford, Connecticut 06107 (US); KRAUS, Nicolas, Enfield, Connecticut 06082 (US); REPP, Timothy C., Barkhamsted, Connecticut 06063 (US)
(74) Representative: Dehns

(56) References cited:
- CN-U- 204 348 277
- JP-A- 2010 233 606
- US-A1- 2019 374 664
- US-A1- 2020 179 543
- US-B1- 10 842 896

## Description

### Technical Field

The present disclosure to directed to disinfecting apparatus and methods that utilize a light source to destroy one or more pathogens, and in particular to such apparatus and methods configured to provide operational information.

### Background Information

Ultraviolet ("UV") light includes light at wavelengths in the band of about 10nm to about 400nm. Within the UV light band are several sub-bands of light typically referred to as "UVA", "UVB", and "UVC". The UVC light sub-band is typically referred to as being about 100nm to about 280nm. Some UVC wavelengths are known to produce germicidal effects on certain harmful microorganisms (e.g., bacterial viruses, mold, and other pathogens) when the aforesaid microorganisms are exposed to the UVC wavelengths at an adequate intensity for a sufficient duration. For example, UVC light may be used to disinfect food and water products. The UVC light may be absorbed by one or more constituents (e.g., proteins, DNA molecules, or the like) within the microorganism, causing the constituent to morph into a form where the ability of the microorganism to replicate or perform other vital biologic functions is negatively affected, and/or is destroyed.

Handheld devices such as smart phones are ubiquitous today and are notorious transporters of microorganisms. Studies have indicated that people check their phone as many as seventeen times a day, and that phones have been found to carry over seventeen thousand bacterial gene copies. Clearly, there is a need for an apparatus that can disinfect a handheld device, including one that can disinfect a handheld device having sensitive electronics such as a smart phone without detriment to the electronics. This has been particularly true during the Covid-19 pandemic. In addition, there is a need for an apparatus that can disinfect a handheld device that permits performance monitoring and additional functionality. US 2019/374664 A1, US 10 842 896 B1, CN 204 348 277 U disclose apparatuses to disinfect handheld devices.

### SUMMARY

The present invention is directed to an apparatus according to present claim 1 and to a method according to present claim 6. The present disclosure encompasses aspects which are not necessarily part of the invention. According to an aspect of the present disclosure, an apparatus for disinfecting a handheld electronic device is provided. The apparatus includes an enclosure, one or more light sources, a disinfecting chamber, a display device, and a controller. The one or more light sources are disposed within the enclosure. The light sources configured to emit one or more wavelengths of light in the UVC sub-band of light. The disinfecting chamber has an interior chamber configured to receive a handheld electronic device, and is configured to receive the one or more wavelengths of light within the interior chamber. The display device is configured to display information. The controller is in communication with the at least one or more light sources, the display device, and a non-transitory memory storing instructions, which instructions when executed cause the controller to: a) control the one or more light sources to emit the one or more wavelengths of light in the UVC sub-band of light for a period of time sufficient to produce a germicidal effect on one or more microorganisms; and b) control the display device to display information during at least the period of time the one or more light sources are controlled to emit the one or more wavelengths of light.

In any of the aspects or embodiments described above and herein, the apparatus may further include a device elevator system configured to move the handheld electronic device within the interior chamber along a lengthwise axis of the disinfecting chamber relative to the one or more light sources. The instructions when executed cause the controller to control the device elevator system to move the handheld electronic device within the interior chamber relative to the one or more light sources a distance sufficient for substantially all of a length of the handheld device to be interrogated with the one or more wavelengths of light emitted from the one or more light sources.

In any of the aspects or embodiments described above and herein, the one or more light sources may include a first light source module in communication with a first side of the disinfecting chamber, and a second light source module in communication with a second side of the disinfecting chamber, wherein the first side of the disinfecting chamber is opposite the second side of the disinfecting chamber and the interior chamber is disposed therebetween.

In any of the aspects or embodiments described above and herein, the first and second light source modules may each include a plurality of light emitting diodes (LED), and each LED is configured to selectively produce at least one of the one or more wavelengths of light in the UVC sub-band of light.

**In** any of the aspects or embodiments described above and herein, the apparatus may include a cooling system configured to move ambient air through an interior region of the enclosure. The instructions when executed may cause the controller to control the cooling system to selectively move ambient air through the interior region of the chamber.

**In** any of the aspects or embodiments described above and herein, the cooling system may include one or more temperature sensors that produce a signal and the instructions when executed cause the controller to control the cooling system to selectively move ambient air through the interior region of the chamber based at least in part on the signals from the one or more temperature sensors.

**In** any of the aspects or embodiments described above and herein, the enclosure may include an enclosure door that is positionable in a closed position and an open position, and in the closed position the enclosure defines an interior region of the enclosure. The enclosure door may include a port that is aligned with the disinfecting chamber, such that the handheld electronic device inserted within the port will be aligned with the disinfecting chamber for receipt within the interior chamber of the disinfecting chamber.

In any of the aspects or embodiments described above and herein, the instructions when executed may cause the controller to determine and record operational information relating to the one or more light sources.

In any of the aspects or embodiments described above and herein, the operational information may include a number of operational cycles performed by at least one of the one or more light sources, or a cumulative operating time of the at least one of the one or more light sources, or both.

In any of the aspects or embodiments described above and herein, the instructions when executed may cause the controller to communicate the operational information to a remote monitoring station.

In any of the aspects or embodiments described above and herein, the instructions when executed may cause the controller to control the display device to display non-operational information during at least the period of time.

In any of the aspects or embodiments described above and herein, the controller may be in communication with a remote source that selectively provides the non-operational information.

In any of the aspects or embodiments described above and herein, the non-operational information may include third-party advertisements.

According to an aspect of the present disclosure, a method of disinfecting a handheld electronic device is provided. The method includes: a) providing an apparatus having an enclosure, one or more light sources disposed within the enclosure, each light source configured to emit one or more wavelengths of light in the UVC sub-band of light, a disinfecting chamber having an interior chamber configured to receive a handheld electronic device, the disinfecting chamber configured to receive the one or more wavelengths of light within the interior chamber, and a display device configured to display information; b) disposing the handheld electronic device within the interior chamber of the disinfecting chamber; c) using the one or more light sources to interrogate the handheld electronic device with the one or more wavelengths of light in the UVC sub-band of light for a period of time sufficient to produce a germicidal effect on one or more microorganisms; and d) controlling the display device to display information during at least the period of time the one or more light sources are used to interrogate the handheld device with the one or more wavelengths of light.

In any of the aspects or embodiments described above and herein, the method may further include using a device elevator system to move the handheld electronic device within the interior chamber along a lengthwise axis of the disinfecting chamber relative to the one or more light sources a distance sufficient for substantially all of a length of the handheld device to be interrogated with the one or more wavelengths of light emitted from the one or more light sources.

In any of the aspects or embodiments described above and herein, the method may further include determining and recording operational information relating to the one or more light sources, the operational information including a number of operational cycles performed by at least one of the one or more light sources, or a cumulative operating time of the at least one of the one or more light sources, or both.

In any of the aspects or embodiments described above and herein, the method may further include controlling the display device to display non-operational information, and/or may include communicating with a remote source that selectively provides the non-operational information.

In any of the aspects or embodiments described above and herein, the non-operational information may include advertising information.

In any of the aspects or embodiments described above and herein, the apparatus is controlled by a custodial party and the custodial party may provide the non-operational information displayed.

The foregoing features and elements may be combined in various combinations without exclusivity, unless expressly indicated otherwise. These features and elements as well as the operation thereof will become more apparent in light of the following description and the accompanying drawings. It should be understood, however, the following description and drawings are intended to be exemplary in nature and non-limiting.

### BRIEF DESCRIPTION OF THE DRAWINGS

FIG. 1 is a perspective view showing an apparatus embodiment mounted on a pedestal.
FIG. 2 is a perspective view of the apparatus embodiment shown in FIG. 1 with the enclosure open.
FIG. 3 is an exploded view of the apparatus embodiment shown in FIGS. 1 and 2.
FIG. 4 is an exploded view of the apparatus embodiment.
FIG. 5 is a perspective view of an apparatus embodiment configured for attachment to a surface, illustrating a handheld electronic device (e.g., a smart phone) partially inserted into the apparatus.
FIG. 6 is a perspective view of a light source module embodiment.
FIG. 7 is a block diagram illustrating an example of communication paths between the various components of the apparatus.
FIG. 8 is a flow chart illustrating an embodiment of operational method of the present disclosure.

### DETAILED DISCLOSURE

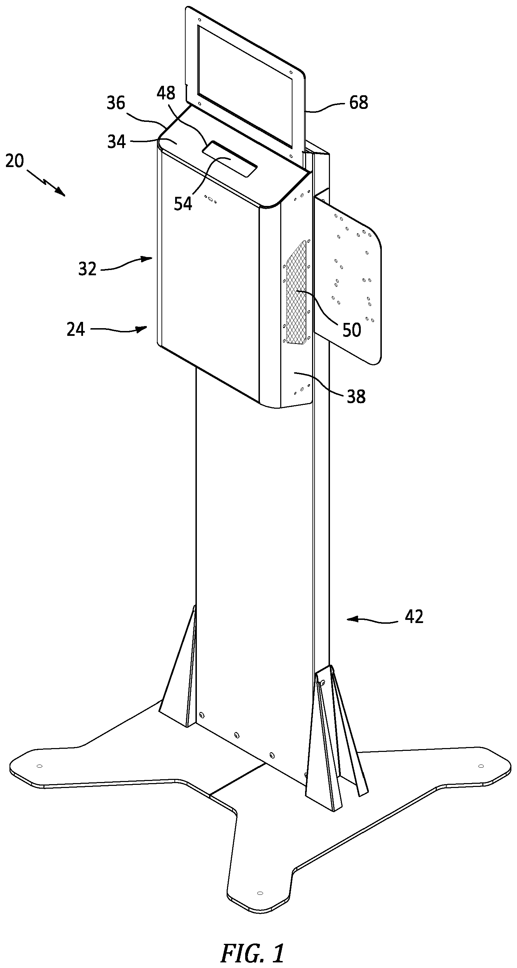

Referring to FIGS. 1-7, the present disclosure is directed to an apparatus 20 and method for disinfecting handheld electronic devices 22. The disinfecting process utilizes UV light at wavelengths that produce germicidal effects on certain harmful microorganisms (e.g., bacterial viruses, mold, and other pathogens). Embodiments of the present disclosure apparatus 20 include an enclosure 24, one or more light sources 26, a disinfecting chamber 28, and a controller 30 that operate in concert to selectively subject surfaces of a handheld electronic device 22 to the aforesaid UV light to produce desired germicidal effects. As will be described herein, aspects of the present disclosure may also include the collection of data relating to the operation of the apparatus 20 that may be used to enhance the reliability and performance of the apparatus 20, and may also include means for providing information to users of the apparatus 20.

The enclosure 24 includes an enclosure door 32 having a top panel 34, a first side panel 36, a second side panel 38 opposite the first side panel 36, and a front panel 40. The top panel 34, first and second side panels 36, 38, and the front panel 40 may be attached to one another to form a unitary door 32. In those embodiments that include a pedestal 42, the door 32 may be pivotally mounted to the pedestal 42 or to an enclosure back panel 44. In those apparatus 20 embodiments that are configured to be mounted to a surface, the door 32 may be pivotally mounted to the enclosure back panel 44 or otherwise pivotally attached. The door 32 is positionable (e.g., pivotable) between a closed position and an open position, and in the open position components of the apparatus 20 are accessible. In the closed position, the door 32 defines an interior region 46 of the enclosure 24. The present disclosure is not limited to an enclosure 24 having a door 32 that is pivotally mounted; e.g., the door 32 may be a unitary component that can be removed to gain access to the enclosure interior region 46. In the embodiments shown in FIGS. 1-5, the top panel 34 of the enclosure door 32 includes a port 48 for receiving a handheld electronic device 22. The present disclosure is not limited to having a port 48 disposed in the top panel 34. In alternative embodiments, a port 48 for receiving a handheld electronic device 22 may be disposed in a side panel 36, 38 or the front panel 40. In some embodiments, the apparatus 20 may include one or more cooling vents 50. In the embodiment shown in FIGS. 1-5, cooling vents 50 are disposed in the side panels 36, 38. In some embodiments, one or more cooling vents 50 may be disposed in locations such as in a front panel 40, a back panel 44, or in a bottom panel 52 as an alternative to being disposed in a side panel 36, 38, or in addition to a cooling vent 50 disposed in a side panel 36, 38.

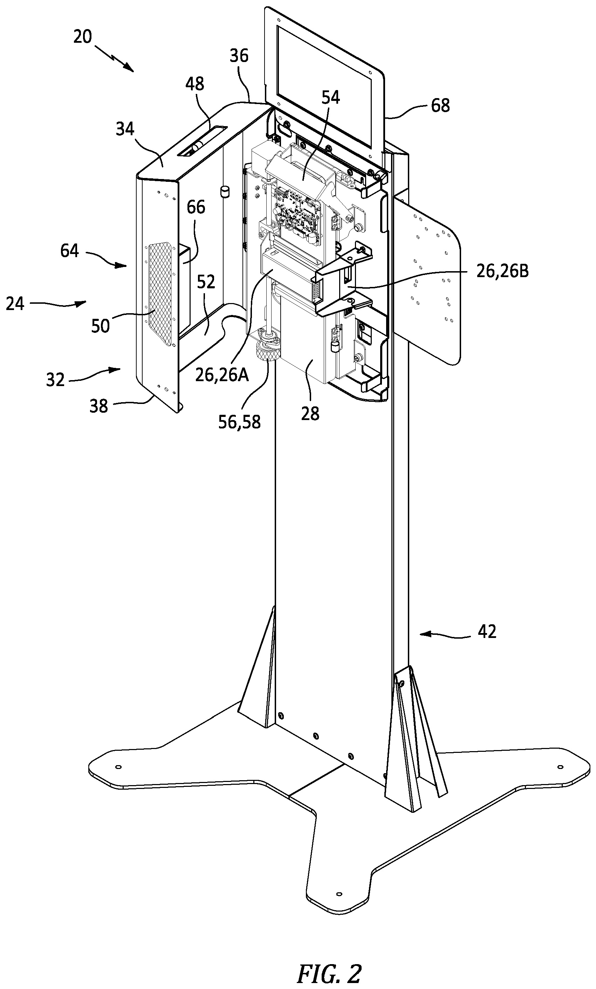

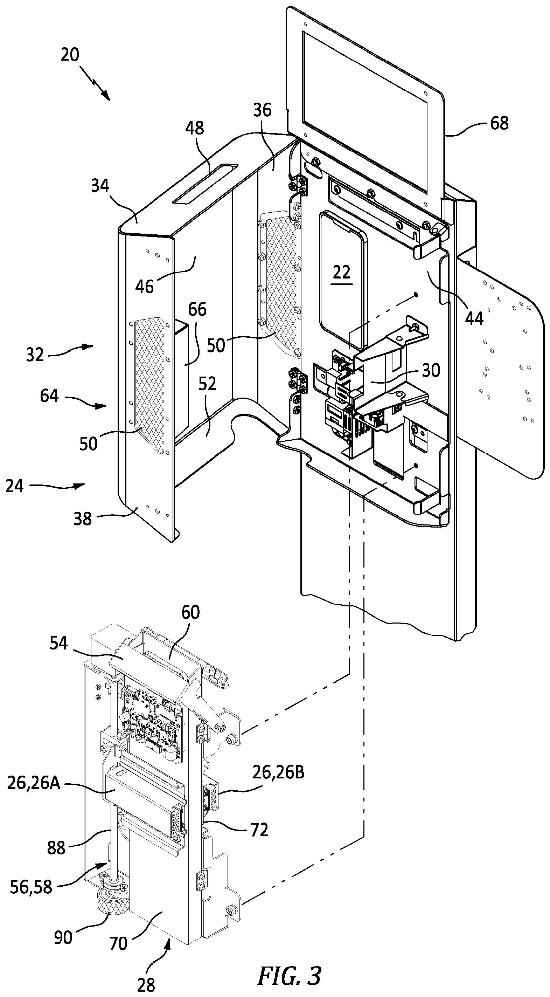

In some embodiments, the apparatus 20 or the enclosure 24 may include a port door 54 that can be translated (or rotated) from a closed door position where the door 54 covers or blocks the port 48 to an open door position where the port door 54 does not cover or block the port 48 and thereby allows a handheld electronic device 22 to be inserted into the enclosure 24. FIG. 1 illustrates an apparatus 20 with a port door 54 disposed in the closed door position and FIGS. 2 and 3 illustrates an apparatus 20 with a port door 54 disposed in the open door position. The port door 54 may be configured for manual movement or automated movement (e.g., movement by mechanical means). The apparatus 20 may be configured so that the port door 54 assumes a default closed door position when the apparatus 20 is not operating and when the apparatus 20 is operating with a handheld electronic device 22 loaded. The apparatus 20 may be configured such that the port door 54 assumes the open door configuration when a device 22 is to be loaded, when it is being loaded, and when a handheld electronic device 22 is being ejected subsequent to being sanitized. In the closed door position, the port door 54 blocks the light produced by the light source 26 from escaping the enclosure 24 to avoid a user being interrogated with such light. The apparatus 20 is not limited to any particular port door 54 actuation mechanism. Automated port door 54 actuation mechanisms may include positional limit switches and the like in communication with other apparatus 20 components such as the handheld electronic device conveyor 56 (e.g., an elevator system 58). An automated port door 54 mechanism may be operated as a result of an input command; e.g., a user touching an input device such as a touch screen or an "on" button or the like. In some embodiments, an automated port door 54 mechanism may be configured to open based on input from a sensor such as a proximity switch, a motion sensor, or the like that senses the presence of a user in close proximity to the apparatus 20. The present disclosure is not limited to including an automated port door 54. For example, in some embodiments, a port door 54 in the form of a pliable member or members may be disposed proximate the enclosure port, which member(s) deflect to allow a handheld electronic device 22 to be inserted and removed from the apparatus 20 and assume a closed door position that blocks light when the apparatus 20 is not in use or when a handheld device is fully inserted into the apparatus 20.

The one or more light sources 26 are configured to produce wavelengths of light within the UVC sub-band; i.e., wavelengths in a nominal range of about 100nm to about 280nm. Light sources 26 that produce wavelengths of light in a nominal range between about 222nm and 270nm are understood to be particularly useful. A light source 26 that produces light at a nominal wavelength of 265nm is a specific example having particular germicidal utility. A non-limiting example of a light source 26 that may be used in the present apparatus 20 is a light emitting diode (LED) that is configured to produce the aforesaid wavelengths; e.g., LEDs that produce light in the range of about 260nm to about 270nm, with a nominal wavelength of 265nm. The present disclosure is not limited to using LEDs or any other particular light source; e.g., a broader band light source in combination with filters that permit passage of the aforesaid wavelengths may be used in some embodiments. As will be described herein, the one or more light sources 26 may include a plurality of light source modules 26A, 26B.

The disinfection chamber 28 may be disposed internally within the apparatus 20 and defines an interior chamber 60 configured to receive the handheld electronic device 22. In some embodiments, the disinfection chamber 28 may be configured to locate the one or more light sources 26 such that light produced by the light sources 26 will interrogate surfaces of the handheld electronic device 22 when the handheld electronic device 22 is received within the interior chamber 60 of the disinfection chamber 28. In some embodiments, the disinfection chamber 28 may be configured to substantially prevent any interrogating light from exiting the interior chamber 60 and may include reflective surfaces disposed relative to the interior chamber 60 to promote the light interrogation of the handheld electronic device 22 surfaces when the device is disposed in the interior chamber 60.

The controller 30 is in communication with the light source 26 and other components to control and/or receive signals therefrom to perform the functions described herein. The controller 30 may include any type of computing device, computational circuit, processor(s), CPU, computer, or the like (collectively referred to hereinafter as a processor) capable of executing a series of instructions that are stored in memory. The controller 30 may have a single processor or multiple processors. Components (e.g., display device 68) of the apparatus 20 may include one or more processors that are in communication with a controller processor. The instructions may include an operating system, and/or executable software modules such as program files, system data, buffers, drivers, utilities, and the like. The executable instructions may apply to any functionality described herein. The controller 30 may include a single memory device or a plurality of memory devices. The present disclosure is not limited to any particular type of memory device, and may include non-transitory memory, read-only memory, random access memory, volatile memory, non-volatile memory, static memory, dynamic memory, flash memory, cache memory, and/or any device that stores digital information. Components within the apparatus 20 may include a memory device that is directly or indirectly in communication with a controller processor. The controller 30 may include, or may be in communication with, an input device that enables a user to enter data and/or instructions, and may include, or be in communication with, an output device configured, for example to display information (e.g., a visual display or a printer), etc. A person of skill in the art will recognize that portions of the controller 30 may assume various forms (e.g., digital signal processor, analog device, etc.) capable of performing the functions described herein. The controller 30 and other apparatus components may be in communication with one another by wired or wireless connection, or any combination thereof.

Some embodiments of the present disclosure apparatus 20 include an internal power supply 62 configured for connection to an external power supply; e.g., 80-264Vac AC power. The present disclosure apparatus 20 is not limited to including an internal power supply 62; e.g., some embodiments may utilize an external power supply. In addition, some embodiments may be configured to utilize a stored electrical energy source such as a battery or the like as the sole power source for the apparatus 20, or in combination with a power source connectable to an external power supply.

Some embodiments of the present disclosure may include a conveyor 56 controllable to move a handheld electronic device 22 relative to the one or more light sources 26 to facilitate exposing the entirety of the handheld electronic device 22 to interrogating light from the light sources 26. As will be described herein, in some embodiments the conveyor 56 may be configured as a device elevator system 58 that is configured to move a handheld electronic device 22 along an axis (e.g., a vertical axis aligned with a gravitational vector) relative to one or more stationary light sources 26. In alternative embodiments, the apparatus 20 may be configured such that the handheld electronic device 22 and the one or more light sources 26 are both translatable relative to one another to cause the entirety of the handheld electronic device 22 to be exposed to interrogating light, or the apparatus 20 may be configured so that the one or more light sources 26 are translatable relative to a stationary handheld electronic device 22. The present disclosure does not require a conveyor 56 or a configuration wherein one or both of the handheld electronic device 22 and the light sources 26 are moved relative to one another; e.g., the apparatus 20 may be configured with light sources 26 that interrogate the entirety of a device 22 held in a stationary position within the disinfection chamber 28.

In some embodiments, the present disclosure apparatus 20 may include a cooling system 64 for cooling apparatus components disposed within the enclosure 24. The cooling system 64 may include a means of driving ambient air through the enclosure 24 such as a fan 66. The cooling system 64 may be configured to operate in a variety of different ways; e.g., the cooling system 64 automatically engages when a device 22 is being sanitized, or engages when the air temperature within the enclosure 24 exceeds a predetermined value, or engages when the temperature of a component disposed within the enclosure 24 exceeds a predetermined value, or the like. For example, the controller 30 may be configured (via stored instructions) to activate a cooling fan 66 as soon as the light source modules 26A, 26B are activated, or if the light sources 26A, 26B have been in an on-mode for predetermined percentage of time (e.g., light sources 26 have cycled "X" number of times in the last "Y" minutes), or the ambient air is above "Z" temperature, etc., and/or until the sensed air temperature within the enclosure 24 (or the temperature of a component) is below a predetermined temperature. The above examples are provided to illustrate modes of cooling system 64 operation and the present disclosure is not limited thereto. Embodiments of the present disclosure utilize a cooling system 64. It is envisioned, however, that some present disclosure apparatus embodiments may not require a cooling system; e.g., apparatus embodiments that utilize a light source that produces minima thermal load. In those embodiments that include a cooling system 64, the present disclosure is not limited to any particular cooling system 64 or cooling system mode of operation.

Embodiments of the present disclosure may be configured to collect and produce information regarding the operation of the apparatus 20, and/or to permit monitoring of the operation of the apparatus 20. For example, some embodiments of the present disclosure may be configured to record the number of operational cycles performed by the apparatus 20 or the cumulative "time-on". In a more specific example, the apparatus 20 may be configured to record the number of operational cycles performed by a light source 26, or the cumulative time the light source 26 is powered to produce light. Light source 26 elements typically have a limited useful life, and the monitoring may provide information regarding the status of the light source 26 relative to that useful life as a preventative measure. As another example, some embodiments of the present disclosure may be configured to monitor performance of the apparatus 20. Sensor feedback, or component operational values (e.g., voltages and/or currents drawn by a component such as a motor or a fan, etc.) may be monitored during operation of the apparatus 20. The monitoring, and information produced thereby, may be provided at the apparatus 20, or may be transmitted to a remote communication station (e.g., a communication station operated by the manufacturer of the apparatus 20, or by the entity providing the apparatus 20 to employees or the public, or the like), or both. If a monitored sensor feedback value or a component operational value is within an acceptable range, or acceptable relative to a predetermined value, the monitored information indicates the apparatus 20 is functioning properly and the apparatus 20 is permitted to function in its normal course. If the monitored sensor feedback value or component operational value is outside of the acceptable range, or skewed relative to a predetermined value, the monitored information may indicate the apparatus 20 is not functioning within specification, or within user chosen guidelines, or the like. The monitoring information may be used to take corrective action as may be necessary, or to schedule maintenance, or to document compliance, or the like, or any combination thereof.

In some embodiments, the apparatus 20 may include a display device 68 (e.g., a LCD, a LED display, or the like) in communication with the controller 30. The display device 68 may be a touch screen operable to communicate instructions to the controller 30. The display device 68 may include a processor configured to communicate with the controller 30. The display device processor may be configured to communicate (e.g., receive data such as non-operational information) directly from a source remote from the apparatus 20, or may be configured to communicate (e.g., receive data such as non-operational information) with a remote source indirectly through the controller 30. The apparatus 20 may be configured (e.g., via stored instructions) to permit the display device 68 to operate in a variety of different modes. For example, the apparatus 20 may be configured to use the display device 68 to display operational information; e.g., "please insert device", "time until sanitization complete", "device operational or not operational", and the like. In some embodiments, the apparatus 20 may be configured to use the display device 68 to display solely non-operational information, or to display non-operational information in combination with operational information. Examples of non-operational information include, but are not limited to, general news or entertainment information, information relating to the business providing the apparatus 20 (e.g., today's business blurb, employee reminders, business visitors for the day, upcoming special events, and the like), or third party advertising information, and the like. These are nonlimiting examples of information types that the apparatus 20 may be configured to display on the display device 68. The display device 68 may be configured to produce both visual information and audio information. The apparatus 20 may be hard wire connected or wirelessly connected to a source (e.g., a remote source) of the information to be displayed, or the apparatus 20 may be configured to permit such information to be downloaded and stored within a memory device of the apparatus 20. In some embodiments, the apparatus 20 may be configured so that the informational content to be displayed may be managed by a remote manager; e.g., the apparatus 20 is in communication with an internet interface that a manager can access that permits the manager to update, change, or modify the informational content. may be particularly useful. The ability of the present disclosure apparatus 20 to operate in multiple different information display modes provides significant advantages and versatility. As stated above, in some embodiments the apparatus 20 may be configured to use the display device 68 to display non-operational information (solely or in combination with operational information) that is controlled by the party providing the apparatus. In some situations, a company (e.g., a custodial party) may elect to utilize the apparatus and its display capability only to display "company" related information. In instances like this, the non-operational information to be displayed can be controlled by the company using the apparatus. In fact, a company using multiple present disclosure apparatus may elect to display different non-operational information in different areas of the company; e.g., a first subset of non-operational information subset management offices, a second subset of non-operational information for manufacturing facilities, a third subset of non-operational information for shipping facilities, and so on. In this manner, a company can tailor the information to the functionality of that portion of the company. In those instances where a company elects to utilize the apparatus and its display capability to display specifically chosen information, the company can control the informational content for appropriateness and messaging. For example, in a retail setting a company providing the apparatus 20 may use the apparatus 20 and its display capability to provide tailored information to the retail consumers (e.g., items on sale, marketing information, etc.). In other instances, a party (company / business / commercial venue, etc.) may elect to utilize the apparatus and its display capability to display third party information; e.g., news, weather information, etc. that will be useful to users of the apparatus and therefore likely reflecting favorably on the party providing the apparatus 20. In other instances, a party may elect to utilize the apparatus 20 and its display capability to display advertising information that can provide a revenue stream directly or indirectly back to be party providing the apparatus. For example, a present disclosure disinfecting apparatus 20 placed in a commercial setting (e.g., a shopping center, a grocery store, etc.) will very likely be exposed to significant human traffic. As a result, it is very likely that the present disclosure disinfecting apparatus 20 not only provides a beneficial disinfecting service, but also provides a desirable advertising platform that advertisers will pay to use.

The apparatus 20 may be configured to display the information continuously, or may be configured to display information selectively. For example, the apparatus 20 may be configured to operate in a display device 68 "sleep mode" in the absence of activity after a predetermined period of time. In the sleep mode, the display device 68 may display a blank screen or a screen saver (e.g., the brand of the apparatus 20, or the name of the business providing the apparatus 20, today's date, etc.). The apparatus 20 may be further configured to transition from the "sleep mode" to an "on mode". The transition from "sleep mode" to "on mode" may be triggered by a variety of different events; e.g., a proximity sensor that senses the presence of a person in close proximity, or a user touching a touch screen type display device 68 (or via other input device), or upon sensing an insertion of a handheld electronic device 22 into the apparatus 20, or via a motion sensor, or the like, or any combination thereof. Once the sanitizing process is completed, the apparatus 20 may be further configured to transition from the "on mode" back to the "sleep mode" after a predetermined period of time in the absence of any triggering event to stay in an "on mode". The present disclosure is not limited to particular mechanism or methodology (e.g., stored instructions) for transitioning from "sleep mode" to "on mode" and vice versa.

As indicated above, the present disclosure apparatus 20 may assume a variety of different configurations. To facilitate a full appreciation of the significance of the present disclosure, nonlimiting examples of present disclosure apparatus 20 embodiments are provided below.

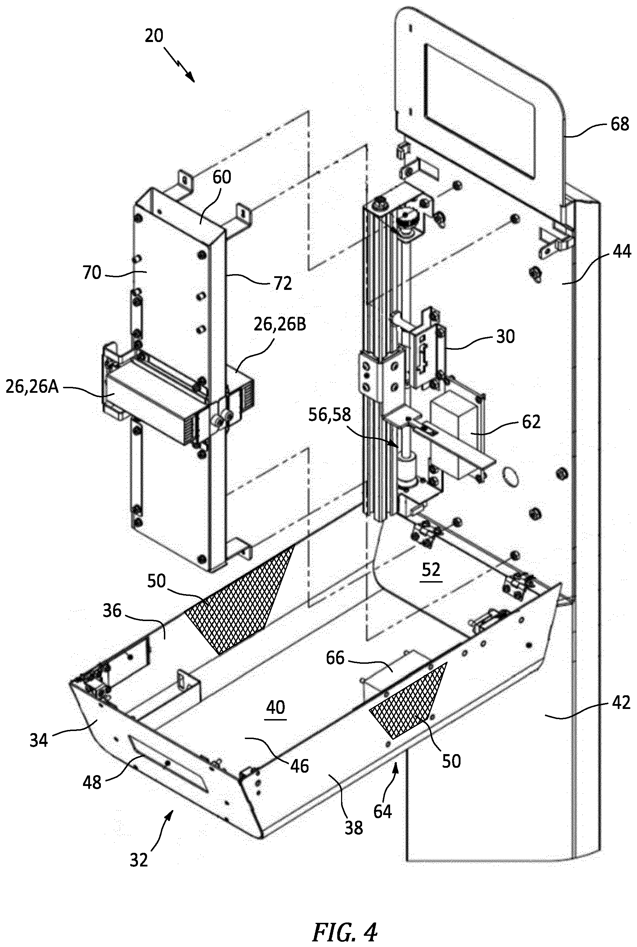

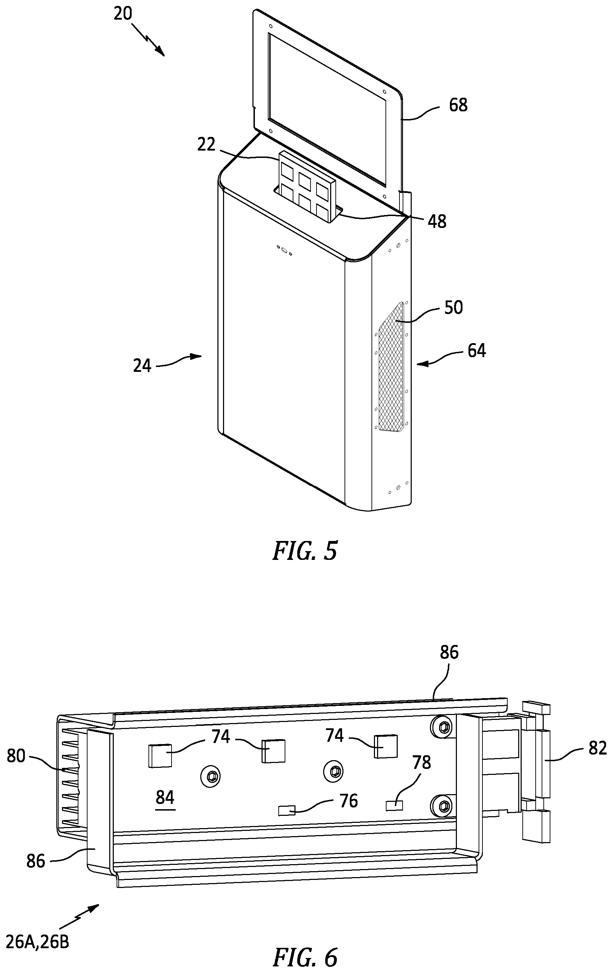

Referring to FIGS. 1-3, an embodiment of the present disclosure apparatus 20 mounted on a pedestal 42 is shown. FIG. 1 is a perspective view of an apparatus 20 embodiment mounted on a pedestal 42 with its enclosure door 32 closed and FIG. 2 illustrates the same embodiment with its enclosure door 32 open. FIG. 3 is an exploded view of the apparatus 20 shown in FIGS. 1 and 2. The apparatus 20 embodiment shown in FIGS. 1-3 includes an enclosure door 32 that is pivotally attached along a side surface panel 36, 38. The apparatus 20 embodiment shown in FIG. 4 includes an enclosure door 32 that is pivotally attached along a bottom panel 52. The pedestal 42 shown in FIGS. 1 and 2 is a nonlimiting example that is configured to facilitate use of the apparatus 20. A pedestal 42 is useful for some embodiments of the present disclosure but is not required. For example, the apparatus 20 embodiment shown in FIG. 5 is configured for attachment to a wall or other surface.

Referring to FIGS. 1-5, embodiments of the apparatus 20 includes an enclosure 24, a cooling system, a disinfection chamber 28, one or more light sources 26, a controller 30, a display device 68, an internal power supply 62, and a conveyor 56 in the form of a device elevator system 58. The enclosure 24 includes an enclosure door 32 having a top panel 34, a first side panel 36, a second side panel 38, a front panel 40, and a bottom panel 52. The top panel 34, first and second side panels 36, 38, front panel 40, and bottom panel 52 are attached to one another to form a unitary door structure. As stated above, the embodiment shown in FIGS. 1-3 has an enclosure door 32 that is pivotally attached along a side surface panel 36 and the embodiment shown in FIG. 4 includes an enclosure door 32 that is pivotally attached along a bottom panel 52. The top panel 34 of these embodiments includes a port 48 for receiving a handheld electronic device 22. FIG. 5 illustrates a handheld electronic device 22 (e.g., a smart phone) partially received within the apparatus 20, with a portion of the device 22 disposed above the port 48.

Referring to FIGS. 1-3, the apparatus 20 includes a port door 54 that is pivotally mounted and can be pivoted between a closed door position where the port door 54 covers the port 48 (e.g., see FIG. 1) and an open door position where the port door 54 does not cover the port 48 (e.g., see FIGS. 2 and 3). In these embodiments, the port door 54 is configured for automated movement (e.g., by mechanical means) in coordination with the device elevator system 58 and the controller 30. In these embodiments, the apparatus 20 is configured such that the port door 54 assumes the open door configuration when a device 22 is to be loaded, when it is being loaded, and when a handheld electronic device 22 is being ejected subsequent to being sanitized, and in the closed door configuration the port door 54 covers the port 48 and blocks substantially all of the light produced by the light source 26 that might otherwise escape from the enclosure 24 during operation or covers the port 48 when the apparatus 20 is not in use to prevent debris / dust entry.

The cooling system 64 includes a cooling fan 66 and one or more cooling vents 50 disposed within the side panels 36, 38 of the enclosure 24. As stated above, the cooling system 64 may be configured to operate in a variety of different ways as described above.

The disinfection chamber 28 is disposed internally within the apparatus 20 and defines an interior chamber 60 configured to receive the handheld electronic device 22. The interior chamber 60 extends along a lengthwise axis of the disinfection chamber 28. The disinfection chamber 28 includes a front panel 40, a back panel 44, and a pair of side panels 36, 38 extending between the front and back panels 40, 44. In the embodiments shown in FIGS. 1-4, as will be detailed below, the one or more light sources 26 include a first light source module 26A and a second light source module 26B. The disinfection chamber 28 is configured to permit the first light source module 26A to be mounted on the front panel 70 and is configured to permit the second light source module 26B to be mounted on the back panel 72. Openings (not shown) in the front and back panels 70, 72 aligned respectively with the first and second light source modules 16A, 26B permit light produced by the light source modules 26A, 26B to enter the interior chamber 60 of the disinfection chamber 28. As stated above, one or more reflective surfaces may be disposed relative to the interior chamber 60 to promote the light interrogation within the interior chamber 60, and the disinfection chamber 28 may be configured to substantially prevent any interrogating light from exiting the interior chamber 60.

In the embodiments shown in FIGS. 1-4 and as indicated above, the one or more light sources 26 include a first light source module 26A and a second light source module 26B mounted on the disinfection chamber 28. Alternative embodiments may include more or less than two light source modules. In the embodiment shown in FIGS. 2-4, the first and second light source modules 26A, 26B are opposite hands of one another. FIG. 6 is a perspective view of a light source module 26A, 26B embodiment that includes a plurality of LEDs 74 each configured to emit light at one or more wavelengths in the UVC sub-band, a processor 76, an electronically erasable programmable read-only memory (EEPROM) 78, a heat sink 80, and a connector 82. The LEDs 74, processor 76, and EEPROM 78 are mounted on a printed circuit board (PCB) 84. The connector 82 is in communication with the PCB 84 and is in direct or indirect communication with an internal power supply 62 and the controller 30. Information may be stored in the EEPROM 78 such as, but not limited to, manufacturer information, model/part number, serial number, operational information, and the like. In regard to operational information, each light source module 26A, 26B may be configured to record LED 74 operational information (e.g., operation cycles, collective operational time, and the like) on the EEPROM 78 or some or all of the operational information may be stored in another memory device (e.g., a memory device associated with the controller 30), or both. The above described light source module 26A, 26B is a nonlimiting example (e.g., a PCB 84 is not required, etc.) and the present disclosure is not limited thereto.

The light source modules 26A, 26B in this example are configured such that the LEDs 74 are oriented to emit light outwardly substantially in a first direction (e.g., inwardly to the interior chamber 60 of the disinfection chamber 28) and the heat sink 80 of each module 26A, 26B is disposed on the opposite side of the LEDs 74. The heat sink 52 is configured to dissipate thermal energy that may be developed by the LEDs 74 during operation. The exemplary light source module 26A, 26B shown in FIG. 5 includes shroud elements 86 configured to enclose the side of the light source module 26A, 26B from which light is emitted relative to the disinfection chamber 28 when the light source module 26A, 26B is attached to the disinfection chamber 28; e.g., around the perimeter of that side of the light source module 26A, 26B. The shroud elements 86 are configured to confine the emitted light and may also be configured to direct cooling air relative to the respective light source module 26A, 26B. Alternatively, shroud elements 86 independent of the respective light source module 26A, 26B may be attached to the disinfection chamber 28 to enclose at least a portion of the respective light source module 26A, 26B. In some embodiments, the light source modules 26A, 26B may be configured to be replaceable units that can be readily attached to the disinfection chamber 28 for use, and readily detached from the disinfection chamber 28 when replacement is necessary or if an alternative light source module 26A, 26B is desired.

Referring to FIGS. 2-4, the apparatus 20 embodiments include a conveyor 56 in the form of a device elevator system 58 that is configured to move a handheld electronic device 22 within the interior chamber 60 of the disinfection chamber 28 along the lengthwise axis of the disinfection chamber 28, and therefore move the handheld electronic device 22 relative to the light source modules 26A, 26B to permit light interrogation of the entirety of the handheld device 22. In these embodiments, the device elevator system 58 moves the handheld electronic device 22 along a vertical axis (i.e., parallel to a gravitational vector). In the specific embodiments shown in FIGS. 2-4, the device elevator system 58 includes a linear actuator 88 driven by a motor 90, and the device elevator system 58 is in communication with the controller 30. As stated above, the present disclosure does not require a conveyor / device elevator system; e.g., in alternative embodiments the apparatus 20 may be configured to interrogate the entirety of a stationary handheld electronic device 22 with UVC light.

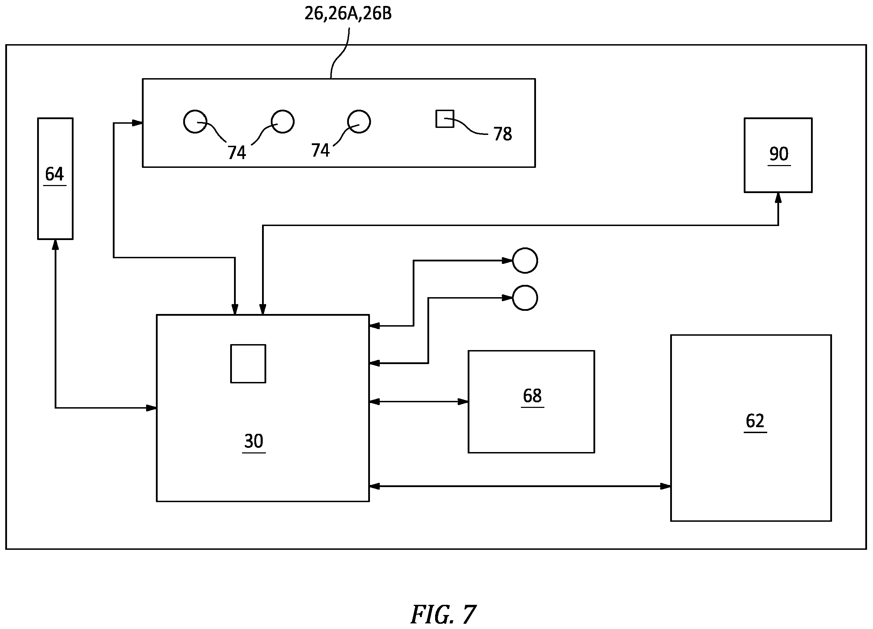

FIG. 7 is a block diagram illustrating communication paths between the various components of the apparatus 20. The controller 30 is shown having a processor 76 in communication with a light source module 26A, 26B, a device elevator system motor 90, a display device 68, a cooling fan 66, and various sensors and limit switches. The light source module 26A, 26B diagrammatically shown in FIG. 6 includes three LEDs 74 and an EEPROM 78. The block diagram shows an internal power supply 62 in communication with the controller 30.

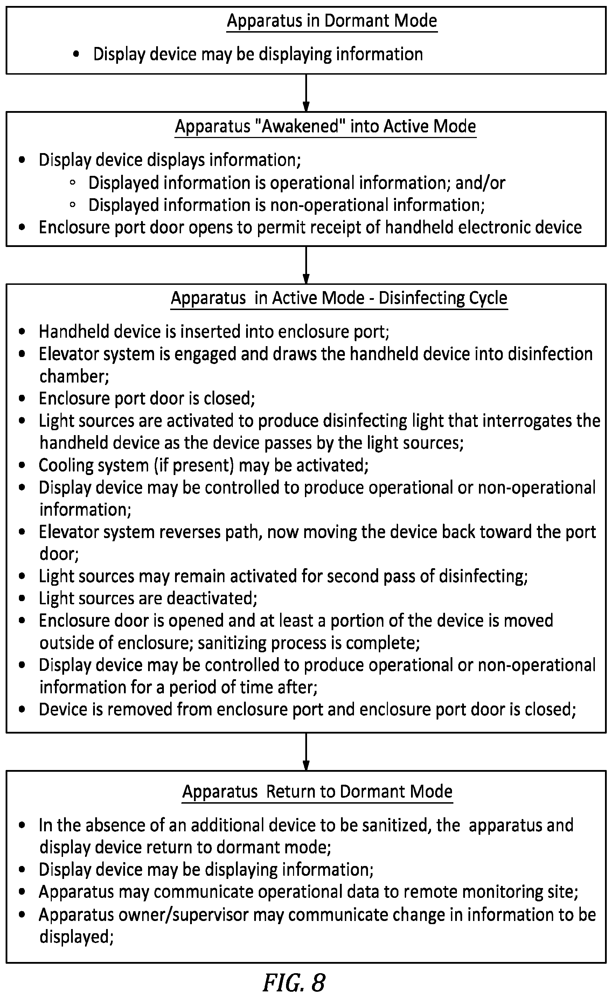

Referring to the flow chart of FIG. 8, a non-limiting example an operation of the present disclosure apparatus 20 and the method associated therewith, is shown. The apparatus 20 may initially be in a dormant mode; e.g., if the apparatus 20 has not been used recently. In the dormant mode, the display device 68 of the apparatus 20 may be controlled to display information. A user engages with the apparatus 20 (either "awakening" via a touch screen, or the presence of the user is sensed (e.g., by a proximity sensor, or a motion sensor, etc.), or by other means) and the apparatus 20 changes into an active mode. Upon entering the active mode, the display device 68 may begin to display information (including audio information in some embodiments) or may change the information being displayed. The apparatus 20 subsequently operates to open the enclosure port door 54 to permit the user to insert the handheld electronic device 22 into the port 48.

The process of receiving the handheld electronic device into the enclosure 24 (and therefore the disinfecting chamber 28) / initiating the disinfection process may be accomplished in a variety of different ways. For example, in some embodiments the apparatus 20 may be configured to draw the device 22 into the enclosure 24 a distance into the enclosure 24 far enough for the port door 54 to be closed before the disinfecting process is initiated. In other embodiments, the apparatus 20 may be configured to draw the device 22 into the enclosure 24 a distance into the enclosure and the disinfecting process may be initiated before the entirety of the device 22 is through the port 48. In either of these modes of operation, the apparatus 20 may sense the presence of the handheld device 22 disposed within the port 48 and automatically draw the device 22 into the enclosure 24, or the apparatus 20 may be configured such that the process of receiving the device 22 into the enclosure begins with a user command (e.g., touch screen input). **In** either of these modes of operation, the port door 54 may be operated to a closed door position (e.g., by mechanical means or automated means) once the device 22 is completely received. Once the disinfecting process is initiated, the device elevator system 58 is actuated to draw the device 22 into or further into the disinfecting chamber 28 and the light sources 26 are activated to produce light at wavelengths that are effective to disinfect the surfaces of the device 22. During the disinfecting process (and/or after) the cooling system 64 (if present) may be activated to pass ambient air through the enclosure 24. The device elevator system 58 operates to move the entirety of the handheld electronic device 22 past the light sources 26. The velocity at which the handheld electronic device 22 traverses past the light source modules 26A, 26B may be chosen to produce a desirable dwell time that is sufficient for complete disinfection of the handheld electronic device 22. **In** some embodiments, the apparatus 20 may include sensors for sensing the geometry of the handheld electronic device 22 to ensure the entirety of the device is traversed past the light source modules 26A, 26B. The device elevator system 58 subsequently operates to reverse the path of the device 22, now moving back toward the enclosure port 48. **In** some embodiments, the light sources 26 may be activated to interrogate the device 22 a second time on the reverse trip.

During the disinfecting process, the controller 30 may be configured collect operational data from one or more apparatus 20 components; e.g., the number of operational cycles performed by a light source module 26 A, 26B and/or a light source's cumulative "time-on", etc. The light sources 26 are subsequently deactivated and the enclosure port door 54 is opened. The device elevator system 58 operates to move at least a portion of the handheld electronic device 22 outside of the enclosure 24 where it can be accessed by the user. The display device 68 may be controlled to continue to display information for some period of time subsequent to the disinfecting process. A period of time after the device is removed from the enclosure 24, and assuming a second device 22 is not inserted, the apparatus 20 may return to a dormant mode.

**In** some embodiments, the apparatus 20 may periodically communicate operational information (e.g., usage data, sensor performance, component operational values such as voltage, current, etc. ) to a remote monitoring site. The collected operational information may then be used to schedule maintenance (e.g., apparatus not functioning, apparatus needs maintenance, X% of useful life of light source module remaining", light source module requires replacement, sensor data out of norm, motor / fan drawing current above norm, etc.). In those instances where the collective recorded information is communicated to the apparatus 20 manufacturer, the aforesaid information can be evaluated over a large population of such apparatus 20 and utilized for performance evaluation, quality control, to determine usage rates, etc.

In addition, periodically, the apparatus 20 owner / supervisor / operator may periodically communicate a change in information to be displayed to the apparatus 20.

While the principles of the disclosure have been described above in connection with specific apparatuses and methods, it is to be clearly understood that this description is made only by way of example and not as limitation on the scope of the disclosure. Specific details are given in the above description to provide a thorough understanding of the embodiments. However, it is understood that the embodiments may be practiced without these specific details.

It is noted that the embodiments may be described as a process which is depicted as a flowchart, a flow diagram, a block diagram, etc. Although any one of these structures may describe the operations as a sequential process, many of the operations can be performed in parallel or concurrently. In addition, the order of the operations may be rearranged. A process may correspond to a method, a function, a procedure, a subroutine, a subprogram, etc.

The singular forms "a," "an," and "the" refer to one or more than one, unless the context clearly dictates otherwise. For example, the term "comprising a specimen" includes single or plural specimens and is considered equivalent to the phrase "comprising at least one specimen." The term "or" refers to a single element of stated alternative elements or a combination of two or more elements unless the context clearly indicates otherwise. As used herein, "comprises" means "includes." Thus, "comprising A or B," means "including A or B, or A and B," without excluding additional elements.

It is noted that various connections are set forth between elements in the present description and drawings (the contents of which are included in this disclosure by way of reference). It is noted that these connections are general and, unless specified otherwise, may be direct or indirect and that this specification is not intended to be limiting in this respect. Any reference to attached, fixed, connected or the like may include permanent, removable, temporary, partial, full and/or any other possible attachment option.

No element, component, or method step in the present disclosure is intended to be dedicated to the public regardless of whether the element, component, or method step is explicitly recited in the claims. As used herein, the terms "comprise", "comprising", or any other variation thereof, are intended to cover a non-exclusive inclusion, such that a process, method, article, or apparatus that comprises a list of elements does not include only those elements but may include other elements not expressly listed or inherent to such process, method, article, or apparatus.

While various inventive aspects, concepts and features of the disclosures may be described and illustrated herein as embodied in combination in the exemplary embodiments, these various aspects, concepts, and features may be used in many alternative embodiments, either individually or in various combinations and sub-combinations thereof.

## Claims

1. An apparatus (20) for disinfecting a handheld electronic device (22), comprising:
an enclosure (24);
one or more light sources (26) disposed within the enclosure, the one or more light sources configured to emit one or more wavelengths of light in the UVC sub-band of light;
a disinfecting chamber (28) having an interior chamber (60) configured to receive a handheld electronic device (22), the disinfecting chamber (28) configured to receive the one or more wavelengths of light within the interior chamber (60);
a display device (68) configured to display information; and
a controller (30) in communication with the at least one or more light sources (26), the display device, and a non-transitory memory storing instructions, which instructions when executed cause the controller (30) to:
control the one or more light sources (26) to emit the one or more wavelengths of light in the UVC sub-band of light for a period of time sufficient to produce a germicidal effect on one or more microorganisms;
control the display device (68) to display information during at least the period of time the one or more light sources (26) are controlled to emit the one or more wavelengths of light;
and
control the display device (68) to display non-operational information during at least the period of time, and the controller (30) is in communication with the remote source that selectively provides the non-operational information;
wherein the non-operational information consists of one or more of: third party advertising information, general news information, entertainment information, or information relating to the business providing the apparatus (20);
wherein the instructions when executed cause the controller (30) to determine and record operational information relating to the one or more light sources (26); and
wherein the operational information includes a number of operational cycles performed by at least one of the one or more light sources (26), or a cumulative operating time of the at least one of the one or more light sources (26), or both.

2. The apparatus (20) of claim 1, further comprising a device elevator system (58) in communication with the controller (30), the device elevator system (58) configured to move the handheld electronic device (22) within the interior chamber (60) along a lengthwise axis of the disinfecting chamber (28) relative to the one or more light sources (26); and
wherein the instructions when executed cause the controller (30) to control the device elevator system (58) to move the handheld electronic device (22) within the interior chamber (60) relative to the one or more light sources (26) a distance sufficient for substantially all of a length of the handheld device (22) to be interrogated with the one or more wavelengths of light emitted from the one or more light sources (26).

3. The apparatus (20) of claim 1 or 2, wherein the one or more light sources (26) include a first light source module (26A) in communication with a first side of the disinfecting chamber (28), and a second light source module (26B) in communication with a second side of the disinfecting chamber (28), wherein the first side of the disinfecting chamber (28) is opposite the second side of the disinfecting chamber (28) and the interior chamber (60) is disposed therebetween.

4. The apparatus (20) of any preceding claim, wherein the enclosure (24) includes an enclosure door (32) that is positionable in a closed position and an open position, and in the closed position the enclosure (24) defines an interior region (46) of the enclosure (24); and
wherein the enclosure door (32) includes a port (48) that is aligned with the disinfecting chamber (28), such that the handheld electronic device (22) inserted within the port (48)will be aligned with the disinfecting chamber (28) for receipt within the interior chamber (60) of the disinfecting chamber (28).

5. The apparatus of any preceding claim, wherein the instructions when executed cause the controller (30) to communicate the operational information to a remote monitoring station.

6. A method of disinfecting a handheld electronic device (22), comprising:
providing an apparatus (20) having an enclosure (24), one or more light sources (26) disposed within the enclosure (24), each light source (26) configured to emit one or more wavelengths of light in the UVC sub-band of light, a disinfecting chamber (28) having an interior chamber (60) configured to receive a handheld electronic device (22), the disinfecting chamber (28) configured to receive the one or more wavelengths of light within the interior chamber (60), and a display device (68) configured to display information;
disposing the handheld electronic device (22) within the interior chamber (60) of the disinfecting chamber (28);
using the one or more light sources (26) to interrogate the handheld electronic device (22) with the one or more wavelengths of light in the UVC sub-band of light for a period of time sufficient to produce a germicidal effect on one or more microorganisms; and
controlling the display device (68) to display information during at least the period of time the one or more light sources (26) are used to interrogate the handheld device (22) with the one or more wavelengths of light;
communicating with a remote source that selectively provides non-operational information and controlling the display device (68) to display the non-operational information;
wherein the non-operational information consists of one or more of: third party advertising information, general news information, entertainment information, or information relating to the business providing the apparatus (20);
further comprising determining and recording operational information relating to the one or more light sources (26), the operational information including a number of operational cycles performed by at least one of the one or more light sources (26), or a cumulative operating time of the at least one of the one or more light sources (26), or both.

7. The method of claim 6, further comprising using a device elevator system (58) to move the handheld electronic device (22) within the interior chamber (60) along a lengthwise axis of the disinfecting chamber (28) relative to the one or more light sources (26) a distance sufficient for substantially all of a length of the handheld device (22) to be interrogated with the one or more wavelengths of light emitted from the one or more light sources (26).

8. The method of claim 6 or 7, wherein the apparatus (20) is controlled by a custodial party and the custodial party provides the non-operational information displayed.

## Patentansprüche

1. Einrichtung (20) zum Desinfizieren einer tragbaren elektronischen Vorrichtung (22), umfassend:
eine Umhüllung (24);
eine oder mehrere Lichtquellen (26), die in der Umhüllung angeordnet sind, wobei die eine oder mehreren Lichtquellen konfiguriert sind, um eine oder mehrere Wellenlängen von Licht im UVC-Teilband von Licht zu emittieren;
eine Desinfektionskammer (28), die eine innere Kammer (60) aufweist, die konfiguriert ist, um eine tragbare elektronische Vorrichtung (22) aufzunehmen, wobei die Desinfektionskammer (28) konfiguriert ist, um die eine oder mehreren Wellenlängen von Licht in der inneren Kammer (60) zu empfangen;
eine Anzeigevorrichtung (68), die konfiguriert ist, um Informationen anzuzeigen; und
eine Steuereinheit (30) in Kommunikation mit der mindestens einen oder mehreren Lichtquellen (26), der Anzeigevorrichtung und einem nichtflüchtigen Speicher, der Anweisungen speichert, wobei die Anweisungen, wenn sie ausgeführt werden, die Steuereinheit (30) veranlassen zum:
Steuern der einen oder mehreren Lichtquellen (26) zum Emittieren der einen oder mehreren Wellenlängen von Licht im UVC-Teilband von Licht für einen Zeitraum, der ausreicht, um eine keimtötende Wirkung auf einen oder mehrere Mikroorganismen zu erzeugen;
Steuern der Anzeigevorrichtung (68), um Informationen während mindestens des Zeitraums anzuzeigen, während dem die eine oder mehreren Lichtquellen (26) zum Emittieren der einen oder mehreren Wellenlängen von Licht gesteuert werden; und
Steuern der Anzeigevorrichtung (68), um Nichtbetriebsinformationen während mindestens des Zeitraums anzuzeigen, und die Steuereinheit (30) ist in Kommunikation mit der entfernten Quelle, die selektiv die Nichtbetriebsinformationen bereitstellt;
wobei die Nichtbetriebsinformationen aus einem oder mehreren bestehen aus: Werbeinformationen Dritter, allgemeinen Nachrichteninformationen, Unterhaltungsinformationen oder Informationen bezüglich des Unternehmens, das die Einrichtung (20) bereitstellt;
wobei die Anweisungen, wenn sie ausgeführt werden, die Steuereinheit (30) veranlassen, Betriebsinformationen bezüglich der einen oder mehreren Lichtquellen (26) zu bestimmen und aufzuzeichnen; und
wobei die Betriebsinformationen eine Anzahl von Betriebszyklen, die von mindestens einer der einen oder mehreren Lichtquellen (26) durchgeführt werden, oder eine kumulative Betriebszeit der mindestens einen der einen oder mehreren Lichtquellen (26) oder beides einschließen.

2. Einrichtung (20) nach Anspruch 1, weiter umfassend ein Vorrichtungsanhebesystem (58) in Kommunikation mit der Steuereinheit (30), wobei das Vorrichtungsanhebesystem (58) konfiguriert ist, um die tragbare elektronische Vorrichtung (22) in der inneren Kammer (60) in Bezug auf die eine oder mehreren Lichtquellen (26) entlang einer längslaufenden Achse der Desinfektionskammer (28) zu bewegen; und
wobei die Anweisungen, wenn sie ausgeführt werden, die Steuereinheit (30) veranlassen, das Vorrichtungsanhebesystem (58) zum Bewegen der tragbaren elektronischen Vorrichtung (22) in der inneren Kammer (60) in Bezug auf die eine oder mehreren Lichtquellen (26) eine Strecke zu bewegen, die ausreicht, damit im Wesentlichen eine gesamte Länge der tragbaren Vorrichtung (22) mit der einen oder den mehreren Wellenlängen von Licht abgefragt wird, das von der einen oder den mehreren Lichtquellen (26) emittiert wird.

3. Einrichtung (20) nach Anspruch 1 oder 2, wobei die eine oder mehreren Lichtquellen (26) ein erstes Lichtquellenmodul (26A) in Kommunikation mit einer ersten Seite der Desinfektionskammer (28) und ein zweites Lichtquellenmodul (26B) in Kommunikation mit einer zweiten Seite der Desinfektionskammer (28) einschließen, wobei die erste Seite der Desinfektionskammer (28) der zweiten Seite der Desinfektionskammer (28) gegenüberliegt und die innere Kammer (60) dazwischen angeordnet ist.

4. Einrichtung (20) nach einem vorstehenden Anspruch, wobei die Umhüllung (24) eine Umhüllungstür (32) einschließt, die in einer geschlossenen Position und einer offenen Position positionierbar ist, und die Umhüllung (24) in der geschlossenen Position einen Innenbereich (46) der Umhüllung (24) definiert; und
wobei die Umhüllungstür (32) eine Öffnung (48) einschließt, die mit der Desinfektionskammer (28) derart ausgerichtet ist, dass die in die Öffnung (48) eingefügte tragbare elektronische Vorrichtung (22) mit der Desinfektionskammer (28) zur Aufnahme in der inneren Kammer (60) der Desinfektionskammer (28) ausgerichtet ist.

5. Einrichtung nach einem vorstehenden Anspruch, wobei die Anweisungen, wenn sie ausgeführt werden, die Steuereinheit (30) veranlassen, die Betriebsinformationen an eine entfernte Überwachungsstation zu kommunizieren.

6. Verfahren zum Desinfizieren einer tragbaren elektronischen Vorrichtung (22), umfassend:
Bereitstellen einer Einrichtung (20), die eine Umhüllung (24) aufweist, eine oder mehrere in der Umhüllung (24) angeordnete Lichtquellen (26), wobei jede Lichtquelle (26) konfiguriert ist, um eine oder mehrere Wellenlängen von Licht im UVC-Teilband von Licht zu emittieren, eine Desinfektionskammer (28), die eine innere Kammer (60) aufweist, die konfiguriert ist, um eine tragbare elektronische Vorrichtung (22) aufzunehmen,
wobei die Desinfektionskammer (28) konfiguriert ist, um die eine oder mehreren Wellenlängen von Licht in der inneren Kammer (60) zu empfangen, und eine Anzeigevorrichtung (68), die konfiguriert ist, um Informationen anzuzeigen;
Anordnen der tragbaren elektronischen Vorrichtung (22) in der inneren Kammer (60) der Desinfektionskammer (28);
Verwenden der einen oder mehreren Lichtquellen (26) zum Abfragen der tragbaren elektronischen Vorrichtung (22) mit der einen oder den mehreren Wellenlängen von Licht im UVC-Teilband von Licht für einen Zeitraum, der ausreicht, um eine keimtötende Wirkung auf einen oder mehrere Mikroorganismen zu erzeugen; und
Steuern der Anzeigevorrichtung (68), um Informationen während mindestens des Zeitraums anzuzeigen, während dem die eine oder mehreren Lichtquellen (26) zum Abfragen der tragbaren Vorrichtung (22) mit der einen oder den mehreren Wellenlängen von Licht verwendet werden;
Kommunizieren mit einer entfernten Quelle, die selektiv Nichtbetriebsinformationen bereitstellt, und Steuern der Anzeigevorrichtung (68), um die Nichtbetriebsinformationen anzuzeigen;
wobei die Nichtbetriebsinformationen aus einem oder mehreren bestehen aus: Werbeinformationen
Dritter, allgemeinen Nachrichteninformationen, Unterhaltungsinformationen oder Informationen bezüglich des Unternehmens, das die Einrichtung (20) bereitstellt;
weiter umfassend Bestimmen und Aufzeichnen von Betriebsinformationen bezüglich der einen oder mehreren Lichtquellen (26), wobei die Betriebsinformationen eine Anzahl von Betriebszyklen, die von mindestens einer der einen oder mehreren Lichtquellen (26) durchgeführt werden, oder eine kumulative Betriebszeit der mindestens einen der einen oder mehreren Lichtquellen (26) oder beides einschließen.

7. Verfahren nach Anspruch 6, weiter umfassend Verwenden eines Vorrichtungsanhebesystems (58) zum Bewegen der tragbaren elektronischen Vorrichtung (22) in der inneren Kammer (60) entlang einer längslaufenden Achse der Desinfektionskammer (28) in Bezug auf die eine oder mehreren Lichtquellen (26) eine Strecke, die ausreicht, damit im Wesentlichen eine gesamte Länge der tragbaren Vorrichtung (22) mit der einen oder den mehreren Wellenlängen von Licht abgefragt wird, das von der einen oder den mehreren Lichtquellen (26) emittiert wird.

8. Verfahren nach Anspruch 6 oder 7, wobei die Einrichtung (20) von einem Verwahrer gesteuert wird und der Verwahrer die angezeigten Nichtbetriebsinformationen bereitstellt.

## Revendications

1. Appareil (20) de désinfection de dispositif électronique portable (22), comprenant :
une enceinte (24) ;
une ou plusieurs sources de lumière (26) disposées à l'intérieur de l'enceinte, les une ou plusieurs sources de lumière étant configurées pour émettre une ou plusieurs longueurs d'onde de lumière dans la sous-bande de lumière UVC ;
une chambre de désinfection (28) présentant une chambre intérieure (60) configurée pour recevoir un dispositif électronique portable (22), la chambre de désinfection (28) étant configurée pour recevoir les une ou plusieurs longueurs d'onde de lumière à l'intérieur de la chambre intérieure (60) ;
un dispositif d'affichage (68) configuré pour afficher des informations ; et
un dispositif de commande (30) en communication avec au moins une des une ou plusieurs sources de lumière (26), le dispositif d'affichage, et une mémoire non transitoire stockant des instructions, lesquelles instructions, lorsqu'exécutées, amènent le dispositif de commande (30) à :
commander les une ou plusieurs sources de lumière (26) afin d'émettre les une ou plusieurs longueurs d'onde de lumière dans la sous-bande de lumière UVC pendant une période de temps suffisante pour produire un effet germicide sur un ou plusieurs microorganismes ;
commander le dispositif d'affichage (68) afin d'afficher des informations pendant au moins la période de temps où les une ou plusieurs sources de lumière (26) sont commandées pour émettre les une ou plusieurs longueurs d'onde de lumière ;
et
commander le dispositif d'affichage (68) afin d'afficher des informations non opérationnelles pendant au moins la période de temps, et le dispositif de commande (30) est en communication avec la source distante qui fournit sélectivement les informations non opérationnelles ;
dans lequel les informations non opérationnelles consistent en une ou plusieurs : d'informations publicitaires de tiers, d'informations d'actualités générales, d'informations de divertissement, ou d'informations relatives à l'entreprise fournissant l'appareil (20) ;
dans lequel les instructions, lorsqu'exécutées, amènent le dispositif de commande (30) à déterminer et enregistrer des informations opérationnelles relatives aux une ou plusieurs sources de lumière (26) ; et
dans lequel les informations opérationnelles incluent un nombre de cycles opérationnels réalisés par au moins une des une ou plusieurs sources de lumière (26), ou un temps de fonctionnement cumulé de la au moins une des une ou plusieurs sources de lumière (26), ou des deux.

2. Appareil (20) selon la revendication 1, comprenant en outre un système élévateur de dispositif (58) en communication avec le dispositif de commande (30), le système élévateur de dispositif (58) étant configuré pour déplacer le dispositif électronique portable (22) à l'intérieur de la chambre intérieure (60) le long d'un axe longitudinal de la chambre de désinfection (28) par rapport aux une ou plusieurs sources de lumière (26) ; et
dans lequel les instructions, lorsqu'exécutées, amènent le dispositif de commande (30) à commander le système élévateur de dispositif (58) afin de déplacer le dispositif électronique portable (22) à l'intérieur de la chambre intérieure (60) par rapport aux une ou plusieurs sources de lumière (26) d'une distance suffisante pour que substantiellement toute une longueur du dispositif portable (22) soit interrogée avec les une ou plusieurs longueurs d'onde de lumière émises à partir des une ou plusieurs sources de lumière (26).

3. Appareil (20) selon la revendication 1 ou la revendication 2, dans lequel les une ou plusieurs sources de lumière (26) incluent un premier module de source de lumière (26A) en communication avec un premier côté de la chambre de désinfection (28), et un second module de source de lumière (26B) en communication avec un second côté de la chambre de désinfection (28), dans lequel le premier côté de la chambre de désinfection (28) est opposé au second côté de la chambre de désinfection (28) et la chambre intérieure (60) est disposée entre les deux.

4. Appareil (20) selon une quelconque revendication précédente, dans lequel l'enceinte (24) inclut une porte d'enceinte (32) qui est positionnable dans une position fermée et une position ouverte, et dans la position fermée, l'enceinte (24) définit une région intérieure (46) de l'enceinte (24) ; et
dans lequel la porte d'enceinte (32) inclut un orifice (48) qui est aligné avec la chambre de désinfection (28), de sorte que le dispositif électronique portable (22) inséré à l'intérieur de l'orifice (48) soit aligné avec la chambre de désinfection (28) pour être reçu à l'intérieur de la chambre intérieure (60) de la chambre de désinfection (28).

5. Appareil selon une quelconque revendication précédente, dans lequel les instructions, lorsqu'exécutées, amènent le dispositif de commande (30) à communiquer les informations opérationnelles à une station de surveillance distante.

6. Procédé de désinfection d'un dispositif électronique portable (22), comprenant :
la fourniture d'un appareil (20) présentant une enceinte (24), une ou plusieurs sources de lumière (26) disposées à l'intérieur de l'enceinte (24), chaque source de lumière (26) étant configurée pour émettre une ou plusieurs longueurs d'onde de lumière dans la sous-bande de lumière UVC, une chambre de désinfection (28) présentant une chambre intérieure (60) configurée pour recevoir un dispositif électronique portable (22), la chambre de désinfection (28) étant configurée pour recevoir les une ou plusieurs longueurs d'onde de lumière à l'intérieur de la chambre intérieure (60), et un dispositif d'affichage (68) étant configuré pour afficher des informations ;
la disposition du dispositif électronique portable (22) à l'intérieur de la chambre intérieure (60) de la chambre de désinfection (28) ;
l'utilisation des une ou plusieurs sources de lumière (26) pour interroger le dispositif électronique portatif (22) avec les une ou plusieurs longueurs d'onde de lumière dans la sous-bande de lumière UVC pendant une période de temps suffisante pour produire un effet germicide sur un ou plusieurs microorganismes ; et
la commande du dispositif d'affichage (68) afin d'afficher des informations pendant au moins la période de temps où les une ou plusieurs sources de lumière (26) sont utilisées pour interroger le dispositif portable (22) avec les une ou plusieurs longueurs d'onde de lumière ;
la communication avec une source distante qui fournit sélectivement des informations non opérationnelles et la commande du dispositif d'affichage (68) afin d'afficher les informations non opérationnelles ;
dans lequel les informations non opérationnelles consistent en une ou plusieurs : d'informations publicitaires de tiers, d'informations d'actualités générales, d'informations de divertissement, ou d'informations relatives à l'entreprise fournissant l'appareil (20) ;
comprenant en outre la détermination et l'enregistrement d'informations opérationnelles relatives aux une ou plusieurs sources de lumière (26), les informations opérationnelles incluant un nombre de cycles opérationnels réalisés par au moins une des une ou plusieurs sources de lumière (26), ou un temps de fonctionnement cumulé de la au moins une des une ou plusieurs sources de lumière (26), ou des deux.

7. Procédé selon la revendication 6, comprenant en outre l'utilisation d'un système élévateur de dispositif (58) pour déplacer le dispositif électronique portable (22) à l'intérieur de la chambre intérieure (60) le long d'un axe longitudinal de la chambre de désinfection (28) par rapport aux une ou plusieurs sources de lumière (26) d'une distance suffisante pour que substantiellement toute une longueur du dispositif portable (22) soit interrogée avec les une ou plusieurs longueurs d'onde de lumière émises à partir des une ou plusieurs sources de lumière (26).

8. Procédé selon la revendication 6 ou la revendication 7, dans lequel l'appareil (20) est commandé par une partie en ayant la garde et la partie en ayant la garde fournit les informations non opérationnelles affichées.
